# EUROPEAN PATENT APPLICATION

(11) **EP 1 595 539 A1**
(43) Date of publication of application: **16.11.2005**
(21) Application number: 04709361.2
(22) Date of filing: 09.02.2004
(51) Int. Cl.: A61K 31/232, A61K 31/7056, A61K 38/21, A61P 1/16, A61P 7/06, A61P 31/12, A61P 43/00

(54) **DRUG FOR REDUCING SIDE EFFECTS IN RIBAVIRIN INTERFERON COMBINATION THERAPY**

(30) Priority: 21.02.2003 JP 2003044751
(71) Applicant: MOCHIDA PHARMACEUTICAL CO., LTD., Shinjuku-ku Tokyo 160-8515 (JP)
(72) Inventor: SATA, Michio, Fukuoka-shi, Fukuoka8140002 (JP); OKAMURA, Takashi, Fukuoka-shi, Fukuoka 8130003 (JP); IDE, Tatsuya, Kurume-shi, Fukuoka 8300048 (JP)
(74) Representative: Grünecker, Kinkeldey, Stockmair & Schwanhäusser Anwaltssozietät
(86) International application number: PCT/JP2004/001325
(87) International publication number: WO 2004/073706

(57) **Abstract**

There is provided a drug for reducing side effects, anemia in particular, in combination therapy of chronic hepatitis C with ribavirin and interferon, which contains as the active ingredient at least one member selected from the group consisting of eicosapentaenoic acid (EPA) and pharmaceutically acceptable salts and esters thereof.

## Description

### Technical Field

The present invention relates to a drug for reducing side effects, anemia in particular, in combination therapy of chronic hepatitis C with ribavirin and interferon (hereinafter abbreviated as IFN), which contains as the active ingredient at least one member selected from the group consisting of eicosapentaenoic acid (hereinafter abbreviated as EPA) and pharmaceutically acceptable salts and esters thereof.

### Background Art

Ribavirin / IFN combination therapy, which is aimed for an improvement of viremia in chronic hepatitis C, has been considered as one of standard first-line therapies for chronic hepatitis C on a global scale because of its higher rate of viral clearance compared with that of the conventional therapy for chronic hepatitis C. However, this therapy has been also known for onset of various side effects with high frequency. In particular, anemia develops with a highest frequency (according to a package insert, oligochromemia and erythrocytopenia as side effects develop with frequencies of 63.1% and 51.3%, respectively) and is put at the head of serious side effects. Where the side effects are serious, there is no choice but to reduce or discontinue the dosage of ribavirin and/or IFN, which is one of the factors that restrict the therapy.

For instance, in JP 2002-542202 A, there is disclosed the use of an antioxidant to improve hemolysis, one of the side effects of ribavirin. The document has reported that four-month ribavirin / IFN combination therapy could be performed without a reduction in dose of ribavirin by administrating vitamins C and E in combination (see page 9).

Meanwhile, in conjunction with EPA which is the active ingredient of the drug for reducing side effects according to the present invention, JP 11-239464 A discloses a composition capable of removing a risk factor on exercise in which an n-3 polyunsaturated fatty acid is incorporated as the active ingredient. It is described in the document that the administration of ethyl ester of EPA to a healthy person allowed the onset level of hemolysis, the maximum level of hemolysis, and the end level of hemolysis to be shifted toward a lower osmotic pressure, suggesting the improvement of the erythrocyte membrane in strength. In addition, the document discloses that the purified-fish-oil capsule containing 28% of EPA that had been taken in advance of high-altitude training allowed an increase in erythrocyte deformability, while allowing no such increase in whole blood viscosity as observed in the group of subjects ingesting no fish oil to be observed (see pages 4-7).

Further, there is another report that an increase in resistance to an oxidative hemolysis was found in 16 cases of healthy persons and 12 cases of hypertriglyceridemic subjects after taking fish oil containing 30% of EPA for 8 weeks (Mabile L. et al., "Moderate intake of n-3 fatty acids is associated with stable erythrocyte resistance to oxidative stress in hypertriglyceridemic subjects", Am. J. Clin. Nutr. (U.S.A.), 2001, 74(4), 449-456) (see pages 449-451 and Fig. 1).

However, any report with respect to utility of EPA as a drug for reducing side effects, particularly anemia, in ribavirin / IFN combination therapy for patients with chronic hepatitis C has not been provided as far as the inventors of the present invention know.

The ribavirin / IFN combination therapy of chronic hepatitis C has become a standard first-line therapy of chronic hepatitis C. However, such therapy has a high frequency of causing a side effect, which is one of the factors that restrict the therapy.

### Disclosure of the Invention

Accordingly, an object of the present invention is to provide a drug which reduces side effects found in ribavirin / IFN combination therapy of chronic hepatitis C so as to allow the drug therapy without reducing or discontinuing the dosage of ribavirin and/or IFN.

The inventors of the present invention have intensively studied with respect to a drug which reduces side effects in combination therapy of chronic hepatitis C with ribavirin and IFN so as to allow the drug therapy without reducing or discontinuing the dosage of ribavirin and/or IFN, and finally completed the present invention by finding out that the drug for reducing side effects of the present invention containing EPA as the active ingredient retains the actions described above.

According to an aspect of the present invention, there is provided a drug for reducing side effects in combination therapy of chronic hepatitis C with ribavirin and interferon, characterized by containing as the active ingredient at least one member selected from the group consisting of eicosapentaenoic acid (also referred to as: icosapentaenoic acid) and pharmaceutically acceptable salts and esters thereof. Preferably, the drug for reducing side effects is adapted to reduce anemia as a side effect.

According to another aspect of the present invention, there is provided a therapeutic agent for chronic hepatitis C with reduced side effects containing ribavirin, interferon, and at least one member selected from the group consisting of eicosapentaenoic acid and pharmaceutically acceptable salts and esters thereof.

The present invention also provides a method of reducing the side effects in combination therapy with ribavirin and interferon, characterized by administering a drug containing as the active ingredient at least one member selected from the group consisting of eicosapentaenoic acid and pharmaceutically acceptable salts and esters thereof before, during, and/or after the administration of ribavirin and interferon in combination therapy of chronic hepatitis C with ribavirin and interferon.

### Best Mode for Carrying Out of the Invention

Hereinafter, the present invention will be described in detail.

In this specification, a "drug for reducing side effects in combination therapy with ribavirin and IFN" refers to one having an ability of reducing at least one of various side effects such as are described under the heading "In the case of a combination with IFN α-2b (recombinant)" in the package insert of a ribavirin-containing pharmaceutical preparation which is commercially available (trade name: Rebetol, manufactured by Schering-Plough Corporation). Specific examples of the side effects include, but not limited to: anemia (erythrocytopenia and oligochromemia), leukopenia, agranulocytosis, thrombocytopenia, aplastic anemia, depression, suicidal ideation, hallucination, delusion, stupor, aggressive behavior, severe hepatic dysfunction, shock, gastrointestinal bleeding, dyspnea, sputum increase, cerebral hemorrhage, bladder cancer, and large bowel cancer as serious side effects; other side effects including pyrexia and influenza-like symptoms (fever, general malaise, headache, arthralgia, myalgia, and rigors), psychoneurotic side effects (insomnia, dizziness, nervousness, hypesthesia, sleepiness, anxiety, apathy, impaired concentration, hoarseness, and paresthesia), hypersensitivity (itching, urticaria, and contact dermatitis), side effects on blood (thrombocytopenia, iron metabolism disorder, reticulocytopenia, monocytosis, lymphocytosis, an increase in serum iron level, lymphopenia, leukocytosis, a decrease in serum iron level, ESR enhancement, eosinophilia, basophilia, lymphadenopathy, and thrombocytosis), side effects on liver (bilirubinemia, an increase in LDH level, an increase in γGTP level, urobilinuria, an increase in ALT (GPT) level, an increase in AST (GOT) level, bilirubin level reduction, zinc sulfate turbidity reaction aberration, and bilirubinuria), side effects on kidney (proteinuria, erythrocyturia, pollakiuria, nephric dysfunction, and urinary disturbance), side effects on circulatory organs (palpitation, flush, arrhythmia, edema (limb / face), peripheral ischemia, and hypertension), side effects on digestive organs (inappetence, abdominal pain, nausea / vomiting, diarrhea, stomatitis, constipation, abnormalities in pancreatic enzyme level (pancreatitis), dipsia, pulpitis / periodontitis, gastritis, a sense of flatulence, indigestion, melena, toothache, glossitis, hemorrhoids, and chilitis), side effects on skin (alopecia, rash, eczema, ringworm, xeroderma, seborrhea, erythema, furuncle, dermatitis, and folliculitis), side effects on nerves/muscles (hypertonia, asthenia, rigidity of limbs, glossoplegia, tremor, and neuralgia), side effects on respiratory organs (cough, pharyngitis, rhinitis, epistaxis, and respiratory infection), side effects on eye (ophthalmalgia, retinal microcirculation disturbance such as eyeground hemorrhage / eyeground leukoderma, abnormalities in visual acuity, xerophthalmia, conjunctivitis, and feeling uncomfortable in eye), and others (backache, thyroid gland dysfunction, hyperuricemia, loss of weight, pains in such organs as skin / limbs, feeling hot, dysgeusia, autoantibody production, pectoralgia, hyperglycemia, hypoalbuminemia, an increase in CRP level, tinnitus, fatigue, hidrosis, hyperproteinemia, herpes simplex, urine sugar, dysosmia, hypocholesterolemia, hypoproteinemia, right hypochondriac pain, blood uric acid reduction, hearing loss, and otitis media); as well as the side effects other than the above that have been reported until now with respect to the ribavirin / IFN combination therapy.

The drug for reducing side effects is preferably adapted to reduce the side effects on blood among the above side effects, specifically, anemia (erythrocytopenia and oligochromemia), leukopenia, agranulocytosis, thrombocytopenia and aplastic anemia as serious side effects as well as other side effects on blood (thrombocytopenia, iron metabolism disorder, reticulocytopenia, monocytosis, lymphocytosis, an increase in serum iron level, lymphopenia, leukocytosis, a decrease in serum iron level, ESR enhancement, eosinophilia, basophilia, lymphadenopathy, and thrombocytosis). Particularly preferred is a drug for reducing side effects which reduces anemia.

In this specification, anemia is to be considered as a state in which the number of erythrocytes and/or the level of hemoglobin decrease(s) absolutely. An absolute measurement is difficult in a routine clinical investigation, so that anemia is defined as a state in which the hemoglobin concentration is equal to or less than the lowest limit of a normal range. Even though it is difficult to give a specific numerical value as a basis for assumption of anemia, according to the standards presented by the WHO, an exemplary value is 13.0 g/dL or less for a healthy adult male and 12.0 g/dL or less for a healthy adult female.

Also in this specification, chronic hepatitis is defined as a morbidity in which inflammation of the liver persists or seems to persist for at least 6 months. Chronic hepatitis C is defined as a chronic hepatitis developed in consequence of human hepatitis C virus (HCV) infection.

Finally, ribavirin is a nucleic acid analog compound represented by the following formula, having broad antiviral activities against various RNA and DNA viruses.

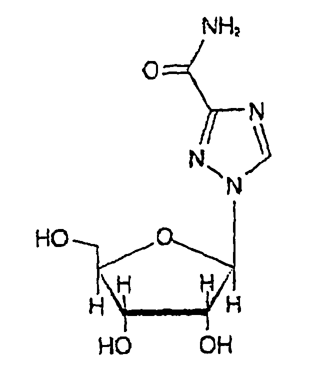

As one of commercially available ribavirin-containing preparations, Rebetol (trade name, a capsulated agent manufactured by Schering-Plough Corp.) is well known. For the purpose of improving viremia in chronic hepatitis C, ribavirin is generally used in combination with IFN and administered at a dose of 400 to 1,600 mg, preferably 600 to 800 mg, a day for an adult. Administration is carried out orally and every day in twice, after morning and evening meals, for 24 weeks. The dosage form, dosage method, frequency of dosage per day and dosage period of ribavirin may be changed appropriately depending on the degree of symptoms, body weight, age, and so forth.

IFNs are proteins having various kinds of bioactivities such as an antiviral action and an antitumor action, and are classified into α, β, and γ types on the basis of differences in their structure and physicochemical properties. In this specification, IFN is not particularly limited and may be any of the IFNs commercially available or under clinical development as long as it is used for the purpose of improving viremia in chronic hepatitis C. Specific examples of the IFN include natural IFN α (IFN α Mochida, manufactured by Mochida Pharmaceutical Co., Ltd.; OIF, manufactured by Otsuka Pharmaceutical Co., Ltd.; or Sumiferon, manufactured by Sumitomo Pharmaceuticals Co., Ltd.), IFN α-2a (Canferon A, manufactured by Takeda Pharmaceutical Co., Ltd.; or Roferon-A manufactured by Chugai Pharmaceutical Co., Ltd.), IFN α-2b (Intron A, manufactured by Schering-Plough Corporation), polyethylene glycol-modified (hereinafter abbreviated as PEGylated) natural IFN α, PEGylated IFN α-2a (Pegasys, manufactured by Chugai Pharmaceutical Co., Ltd.), PEGylated IFN α-2b (PEG-Intron A), natural IFN β (IFN β Mochida, manufactured by Mochida Pharmaceutical Co., Ltd.; or Feron, manufactured by Toray Industries, Inc.), PEGylated natural IFN β, natural IFN γ, consensus IFN (Advaferon, manufactured by Yamanouchi Pharmaceutical Co., Ltd.), PEGylated consensus IFN, and a combination thereof. Natural IFN α, IFN α-2b, PEGylated IFN α-2a, PEGylated IFN α-2b, natural IFN β, and the like are preferable.

In this specification, the dosage amount, dosage period, dosage schedule, dosage route, and so on of IFN are not particularly limited as long as they are those generally employed upon the administration for an improvement of viremia in chronic hepatitis C. Typically, 1 to 10 million IU a day of an interferon is subcutaneously, intramuscularly or intravenously administered at a time or in divided doses every day or intermittently, for instance 3 times a week, for 2 to 48 weeks. The dosage regimen can be appropriately changed depending on the type and amount of a virus, the weight and age of a patient, or the like. Preferably, 6 to 10 million IU a day is administered every day for 2 to 8 weeks, then intermittently for 22 to 46 weeks. Such a regimen, however, may appropriately be changed depending on the kind or dosage form of IFN. In the case of PEGylated IFN α-2a (Pegasys, manufactured by Chugai Pharmaceutical Co., Ltd.) for instance, it is generally possible to subcutaneously administer the interferon once a week, every time at a dose of 180 µg.

EPA to be used in the present invention may be one commercially available or obtained by the purification from fish oil, or EPA-producing bacterial cells and the culture medium thereof using any of known methods such as a continuous distillation method, a urea adduct method, a liquid chromatography, a supercritical fluid chromatography, and a combination thereof. If necessary, the resulting EPA may be subjected to an esterification treatment to make it into an ester such as alkyl ester including ethyl ester, or glyceride. In addition, it may be prepared in the form of a salt, specifically, salt with an inorganic base such as sodium salt and potassium salt, salt with an organic base such as benzylamine salt and diethylamine salt, or salt with a basic amino acid such as arginine salt and lysine salt. In the present invention, EPA includes the above salts and esters in addition to a free fatty acid unless otherwise specified. It is preferred that EPA to be administered to a human or an animal is pharmaceutically acceptable.

The drug for reducing side effects of the present invention may contain other fatty acid as an additional active ingredient together with EPA, not to mention that the pure product of EPA can be used for. Examples of such a fatty acid include: unsaturated fatty acids such as docosahexaenoic acid, docosapentaenoic acid, docosamonoenoic acid, arachidonic acid, eicosatetraenoic acid, eicosatrienoic acid, eicosamonoenoic acid, octadecatetraenoic acid, α-linolenic acid, linoleic acid, oleic acid, palmitoleic acid, hexadecatetraenoic acid, hexadecatrienoic acid, and hexadecadienoic acid; and saturated fatty acids such as behenic acid, arachidic acid, stearic acid, palmitic acid, and myristic acid. The fatty acid exemplified as above may be in the free form, a salt with an inorganic base such as sodium salt, a salt with an organic base such as benzylamine salt, or an ester such as alkyl ester including ethyl ester or glyceride.

EPA used in the drug for reducing side effects of the present invention may comprise 50 wt% or more, preferably 70 wt% or more, more preferably 85 wt% or more of the total of the fatty acids in the drug. It is desirable that the content of arachidonic acid is low. A preferable active ingredient is eicosapentaenoic acid ethyl ester (hereinafter abbreviated as EPA-E). A compound as the active ingredient of the drug for reducing side effects of the present invention may be administered by itself or, alternatively, combined with a conventional carrier or medium which is appropriately selected to provide a preparation suitable for pharmaceutical use, such as excipient, binder, lubricant, coloring agent, flavoring agent; sterilized water or vegetable oil as required; as well as a harmless organic solvent or harmless solubilizer (e.g., glycerin or propylene glycol), emulsifier, suspending agent (e.g., Tween 80 or a gum arabic solution), isotonizing agent, pH adjuster, stabilizer, and soothing agent.

As EPA is of a highly unsaturated nature, it is desirable that the above pharmaceutical preparation further contains an antioxidant in an amount effective enough to prevent the oxidation of EPA. Examples of the antioxidant include butylated hydroxytoluene (BHT), butylated hydroxyanisole (BHA), propyl gallate, gallic acid, pharmaceutically acceptable quinone, and α-tocopherol.

The dosage form of the preparation may be exemplified by tablets, capsules, microcapsules, granules, fine granules, powders, liquid preparations for oral administration, suppositories, syrups, inhalants, eye drops, ointments, injections (emulsifying, suspending, or nonaqueous ones), or solid injections to be emulsified or suspended just prior to use. Administration to a patient may be carried out in an oral, intravenous, intraarterial, inhalational, ophthalmic, rectal, vaginal, or again, external manner. Particularly preferred, however, is the oral administration of the preparation in, for instance, soft capsule or microcapsule form obtained by encapsulation. It is also preferable to intravenously or intraarterially administer the preparation as an injection (emulsifying, suspending or nonaqueous one) or a solid injection to be emulsified or suspended just prior to use.

It should be noted that either of Epadel and Epadel S (both manufactured by Mochida Pharmaceutical Co., Ltd.), each being a high-purity EPA-E-containing soft capsule already commercially available in Japan as a safe therapeutic agent for arteriosclerosis obliterans and hyperlipemia with less side effects, may also be applied.

The drug for reducing side effects of the present invention can be administered at a dose sufficient to exert the action of interest and the dosage amount can be increased or decreased appropriately in consideration of the dosage form, dosage method, frequency of dosage per day, the degree of symptoms, body weight, age, and so on.
In the case of an oral administration, the drug is administered at a dose of 0.1 to 9 g/day, preferably 0.5 to 6 g/day, more preferably 1 to 3 g/day, as EPA in three times. If necessary, however, the entire dose may be administered at a time, or in several times. In the case of an intravenous or intraarterial administration, the drug is administered at a dose of 1 to 200 mg, preferably 5 to 100 mg, more preferably 10 to 50 mg, as EPA at a time or in divided dosages. It is also possible to administer the entire dose continuously over hours to several days using a drip, infusion pump or the like, as required.

EPA can be administered according to any schedule and for any period within a time beginning prior to the dosage period of ribavirin and/or IFN in a standard ribavirin / IFN combination therapy and lasting through the dosage period of ribavirin and/or IFN to lapse after the dosage period of ribavirin and/or IFN. In terms of the prevention of side effects, it is preferable to start administering EPA before the administration of ribavirin / IFN is put into practice. In terms of the reduction in the side effects caused by ribavirin / IFN, the administration of EPA may be started after the administration of ribavirin / IFN. It is preferred that EPA is administered continuously for at least one week in principle, and a more preferable dosage period is from one month to two years.

### Example

Example of the present invention will be described below, although the present invention is in no way restricted to it.

### (Example 1)

### [Subject and Method]

The present study was conducted on 6 patients (5 males and 1 female, aged from 38 to 60) with chronic hepatitis C who had developed anemia due to ribavirin / IFN α combination therapy (males with a hemoglobin level of 14 g/dL or less and females with a hemoglobin level of 11 g/dL or less being considered as anemic in the present example). After the onset of anemia, each patient received 600 mg of EPA-E (trade name: Epadel, manufactured by Mochida Pharmaceutical Co., Ltd.) three times a day by oral administration every after meal for 2 months. Depending on the disease cases, the time of starting the administration of EPA-E varied within the range from 1 to 4 months after the start of administration of ribavirin / IFN α. In the ribavirin / IFN α combination therapy, ribavirin (trade name: Rebetol, manufactured by Schering-Plough Corporation) was orally administered to each patient of over 60 kg in body weight at a dose of 400 mg every after morning meal and every after evening meal as well, and to each patient of 60 kg or less at a dose of 200 mg every after morning meal and 400 mg every after evening meal. Simultaneously, 6 million IU a day of IFN α-2b (trade name: Intron A, manufactured by Schering-Plough Corporation) was intramuscularly administered everyday for two weeks, followed by intermittent administration of three times a week for 22 weeks.

### [Results]

The hemoglobin level was obtained from the individual cases before the ribavirin / IFN combination therapy, before the administration of EPA, after the EPA administration for one month, and after the EPA administration for two months and the respective averages of the obtained values were found. The results are set forth in Table 1.

**Table 1**

| | Hemoglobin level (g/dL) | | | |
|---|---|---|---|---|
| | Before ribavirin/IFN combination therapy | Before EPA administration | After EPA administration for one month | After EPA administration for two months |
| Case 1 (male) | 15.1 | 13.7 | 14.3 | 14.0 |
| Case 2 (female) | 12.4 | 10.8 | 12.1 | 11.7 |
| Case 3 (male) | 15.7 | 10.7 | 11.1 | 11.0 |
| Case 4 (male) | 14.1 | 9.7 | 9.9 | 9.8 |
| Case 5 (male) | 16.5 | 9.1 | 9.6 | 10.3 |
| Case 6 (male) | 16.7 | 10.8 | 11.4 | 12.0 |
| Average | 15.1 | 10.8 | 11.4* | 11.5* |

| | | | | |
|---|---|---|---|---|
| *: p < 0.05 (vs. Before EPA administration) | | | | |

As shown in Table 1, a significant improvement in hemoglobin level was recognized when the administration of EPA was conducted. In addition, in all the six cases, the 24-week drug administration could be completed without reducing or discontinuing the dosage of ribavirin and IFN α.

Furthermore, there were no adverse events which seemed to be induced by the EPA administration.

From the above facts, it is confirmed that EPA can be an effective and useful drug for reducing side effects in ribavirin / IFN combination therapy of chronic hepatitis C.

### Industrial Availability

A drug for reducing side effects in combination therapy of chronic hepatitis C with ribavirin and interferon, containing as the active ingredient at least one member selected from the group consisting of eicosapentaenoic acid and pharmaceutically acceptable salts and esters thereof, allows the drug therapy without reducing or discontinuing the dosage of ribavirin and/or IFN as the drug reduces the side effects found in the therapy.

## Claims

1. A drug for reducing side effects in combination therapy of chronic hepatitis C with ribavirin and interferon, containing as an active ingredient at least one member selected from the group consisting of eicosapentaenoic acid and pharmaceutically acceptable salts and esters thereof.

2. The drug for reducing side effects according to claim 1, wherein the side effects include anemia.

3. The drug for reducing side effects according to claim 1 or 2, wherein the interferon is α-type interferon.

4. A drug containing as an active ingredient at least one member selected from the group consisting of eicosapentaenoic acid and pharmaceutically acceptable salts and esters thereof, which is administered before, during, and/or after the administration of ribavirin and interferon in combination therapy of chronic hepatitis C with ribavirin and interferon so as to reduce side effects in the combination therapy with ribavirin and interferon.

5. A use of at least one member selected from the group consisting of eicosapentaenoic acid and pharmaceutically acceptable salts and esters thereof in a production of a drug for reducing side effects in combination therapy of chronic hepatitis C with ribavirin and interferon.

6. A therapeutic agent for chronic hepatitis C with reduced side effects, containing ribavirin, interferon, and at least one member selected from the group consisting of eicosapentaenoic acid and pharmaceutically acceptable salts and esters thereof.
